# EUROPEAN PATENT APPLICATION

(11) **EP 4 131 275 A1**
(43) Date of publication of application: **08.02.2023**
(21) Application number: 20926771.5
(22) Date of filing: 23.12.2020
(51) Int. Cl.: G16B 35/00

(54) **METHOD AND COMPUTER PROGRAM FOR PREDICTING NEOANTIGEN BY USING PEPTIDE SEQUENCE AND HLA ALLELE SEQUENCE**

(30) Priority: 24.03.2020 KR 20200035815
(71) Applicant: Theragen Bio Co., Ltd., Gyeonggi-do 13488 (KR)
(72) Inventor: HWANG, Taesoon, Seongnam-si Gyeonggi-do 13488 (KR); HONG, Seong-Eui, Seongnam-si Gyeonggi-do 13488 (KR)
(74) Representative: M. Zardi & Co S.A.
(86) International application number: PCT/KR2020/019017
(87) International publication number: WO 2021/194057

(57) **Abstract**

The present disclosure provides a method for predicting a neoantigen by using a peptide sequence and an HLA allele sequence, the method comprising the steps of: receiving a peptide sequence and an HLA allele sequence both extracted from a target cancer tissue as an input; outputting a first prediction value that predicts immunogenicity of the peptide sequence, by acquiring T cell activity data from the peptide sequence and inputting the T cell activity data into an immunogenicity prediction model; outputting a second prediction value that predicts a binding affinity of the peptide sequence and the HLA allele sequence, by acquiring binding data from the HLA allele sequence and inputting the binding data into a binding prediction model; outputting a third prediction value that predicts immune tolerance of the target cancer tissue, by inputting the T cell activity data and the binding data to an immune tolerance prediction model; and generating neoantigen information of a target cell by using the T cell activity data and the first to third prediction value.

## Description

### FIELD OF THE INVENTION

The present invention relates to a method and a computer program of predicting a neoantigen by using a peptide sequence and an HLA allele sequence.

### BACKGROUND OF THE INVENTION

With the development of new anticancer drugs, first generation chemotherapy and second generation targeted agent have been used, and recently, third generation immunotherapy are in the spotlight. In particular, the third generation immunotherapy has advantages of significantly lower side effects because it utilizes a patient's own immune system, unlike previous anticancer drugs. However, despite these advantages, there is a limit in that only patients who show expression of marker genes such as PD-L1 and microsatellite instability (MSI-H) can establish a treatment strategy using the immunotherapy. Due to these restrictions, it is necessary to establish a strategy to treat patients who have difficulty in administering existing anticancer drugs, and one of the alternatives is a cancer vaccine using a neoantigen. Each patient's cancer tissue has mutations that is not found in normal tissues, and the key strategy for cancer vaccines is to use peptides derived from these mutation as neoantigens so that the patient's immune system can recognize and attack the neoantigen. The essential prerequisites of this process are as follows. First, it is necessary to confirm stable binding between the mutant derived peptide and the patient specific HLA allele, and secondly, it is also necessary to confirm whether there is immunogenicity in the mutant derived peptide, which stimulates the patient's immune system well. In particular, in order to reflect the immunogenicity as much as possible, all steps in which immunogenicity occurs should be simulated and key features should be extracted. However, there may be missing steps in this process, and these may be limitations for future immunogenicity prediction.

Accordingly, the present technology embodies a strategy of preventing omission of immune processes and extracting key features thereof based on the combination data of peptide sequences and HLA sequences, HLA in the case of human, which has immunogenicity known to date. In addition, the present technology was intended to derive neoantigens applicable to patients, by modeling a binding affinity of a neoantigen to HLA, an immunogenicity of the peptide sequence itself, and an immune tolerance that has a binding affinity but does not induce immunity. The background art mentioned above is technological information that the inventor possessed to devise the present invention or acquired during devising the present invention and is not a known technology disclosed to the public prior to the filing of the present invention.

### DETAILED DESCRITION OF THE INVENTION

### TECHNICAL PROBLEMS

An object of the present invention is to predict that a mutation-derived peptide sequence present in a patient's cancer tissue binds to a patient specific HLA, goes through a series of immune processes, and finally exhibits immunogenicity and to determine neoantigens that can be used for cancer vaccine customized for cancer patients based on the predictions.

### SUMMARY OF THE INVENTION

According to an embodiment of the present invention, a method of predicting a neoantigen by using a peptide sequence and an HLA allele sequence, may comprise the steps of: receiving a peptide sequence and an HLA allele sequence both extracted from a target cancer tissue as an input; outputting a first prediction value that predicts immunogenicity of the peptide sequence, by acquiring T cell activity data from the peptide sequence and inputting the T cell activity data into an immunogenicity prediction model; outputting a second prediction value that predicts a binding affinity of the peptide sequence and the HLA allele sequence, by acquiring binding data from the HLA allele sequence and inputting the binding data into a binding prediction model; outputting a third prediction value that predicts immune tolerance of the target cancer tissue, by inputting the T cell activity data and the binding data to an immune tolerance prediction model; and generating neoantigen information of a target cell by using the T cell activity data and the first to third prediction value.

At least one of the immunogenicity prediction model, the binding predictive model, and the immune tolerance prediction model may be trained by a machine learning algorithm, based on a training data set including a peptide sequence and an HLA allele sequence present in a plurality of target cancer tissues.

The target cancer tissue may include a cell engineered to express a single HLA class I allele or single HLA class II allele.

The target cancer tissue may include a human cell obtained from or derived from a plurality of patients.

The target cancer tissue may include a fresh or frozen tumor cell obtained from a plurality of patients.

The target cancer tissue may include a fresh or frozen tissue cell obtained from a plurality of patients.

The target cancer tissue may include a peptide identified by using T-cell analysis.

The training data set may include at least one of data related to a proteomic sequence related to the target cancer tissue, data related to an HLA peptide sequence related to the target cancer tissue, binding data between a peptide and an HLA allele that are related to the target cancer tissue; data related to a transcriptome related to the target cancer tissue; and data related to a genome related to the target cancer tissue.

The immunogenicity prediction model may be a model trained with the T cell activity data from the peptide sequence as an input and the immunogenicity of the peptide sequence as an output.

The binding prediction model may be a model trained with the binding data from the HLA allele sequence and the peptide sequence as an input and the binding affinity of the peptide sequence and the HLA allele sequence as an output.

The immune tolerance prediction model may be a model trained with the T cell activity data from the peptide sequence and the HLA allele sequence, and the binding data from the HLA allele sequence and the peptide sequence as an input and the immune tolerance between the peptide sequence and the HLA allele sequence.

A computer program according to an embodiment of the present invention may be stored in a computer-readable storage medium to execute any one method among the methods according to the embodiments of the present invention by using a computer.

In addition to this, another method, another system, and a computer-readable storage medium of recording a computer program for executing the method are further provided to embody the present invention.

Other aspects, features, and advantages other than those described above will become apparent from the following drawings, claims, and detailed description of the present invention.

### TECHNICAL EFFECTS OF THE INVENTION

According to an embodiment of the present invention described above, a method of predicting a neoantigen by using a peptide sequence and an HLA allele sequence, can predict a binding affinity between the peptide sequence and the HLA allele sequence included in the cancer tissue and immunogenicity of the peptide sequence and can determine the neoantigen in the cancer tissue based on the predicted immunogenicity.

### BRIEF DESCRIPTION OF THE DRAWINGS

FIG. 1 is a block diagram of a neoantigen determination device 100 according to embodiments of the present invention.
FIG. 2 is an exemplary graph of type information of HLA class I contained in cells of Koreans.
FIG. 3 is a block diagram of a neoantigen determination device 100 according to embodiments of the present invention.
FIG. 4 is a block diagram for explaining input data and output data of a neoantigen determination device 100.
FIGs. 5 to 7 are diagrams illustrating examples of a neoantigen determination system according to embodiments of the present invention.
FIG. 8 is a block diagram of a learning server 10 for learning an immunogenicity prediction model, a binding prediction model, an immune tolerance prediction model, and the like.
FIG. 9 is an exemplary diagram of an immunogenicity prediction model, a binding prediction model, an immune tolerance prediction model.

### MODES FOR THE INVENTION

Hereinafter, configurations and operations of the present invention will be described in detail with reference to embodiments of the present invention shown in the accompanying drawings.

Since the present invention can be modified to various forms and have various embodiments, specific embodiments are illustrated in the drawings and described in detail in the detailed description. Effects and features of the present invention and a method of achieving them, will become apparent with reference to the embodiments described below in conjunction with the drawings. However, the present invention is not limited to the embodiments disclosed below and may be embodied in various forms.

Hereinafter, embodiments of the present invention will be described in detail with reference to the accompanying drawings, and when described with reference to the drawings, the same or corresponding components are given the same reference numbers, and the description of components that have been already discussed in the previous embodiment will be omitted to avoid redundancy.

In the following embodiments, terms such as first and second are used for the purpose of distinguishing one component from another, without limiting meaning.

In the following examples, singular expression includes plural expression unless the context clearly states otherwise.

In the following embodiments, terms such as "comprise," "include," or "have" indicate that the features or components described in the specification are present and do not exclude the possibilities that one or more other features or components may be added.

In the drawings, the size of components may be exaggerated or reduced for convenience of description. For example, since the size and thickness of each component shown in the drawings are arbitrarily illustrated for convenience of description, the present invention is not necessarily limited to the illustration in the drawings.

In cases where certain embodiments may be embodied in different ways, a specific process may be performed in a different order from the described. For example, two processes described in succession may be performed substantially simultaneously or may be performed in an order opposite to the order described.

In this description, a target cancer tissue refers to a tissue to be tested. For example, the target cancer tissue may be a cancer tissue in which an antigen capable of eliciting an immune response is to be detected. Preferably, the target cancer tissue may be a tumor cell or an aggregate of cancer cells.

Here, mutation indicates all phenomena in which a sequence of nucleotide sequences A (adenine), T (thymine), G (guanine), and C (cytosine) of a gene containing genetic information in each organism, is altered to be different from the original genetic information of a corresponding species. The mutation causes small scale or large scale structural changes. Small scale mutations include a point mutation in which a single nucleotide sequence is converted and mutations in which nucleotide sequences are additionally inserted or deleted. Large scale mutations that occur on a large scale and affect the structure include gene duplication, gene deletion, chromosomal inversion, interstitial deletion, chromosomal translocation, and loss of heterozygosity.

Mutations are largely divided into germline mutations and somatic mutations depending on types of cell in a mutation occurs. Somatic mutation is a gene mutation that occurs in somatic cells, also called somatic cell mutation or somatic modification, and may be caused by a mutation in a gene or a chromosomal abnormality.

Due to the occurrence of such a mutation, the function of the protein produced by the gene may be changed, and a specific function may be lost, or activated as another function. Since these changes in protein function cause or accelerate cancer development, these mutations may be deeply related to cancer development and progression either directly or indirectly.

As described above, nucleotide sequence in DNA containing genetic information of a living organism consists of A, T, G, and C. When these nucleotide sequences are gathered three in a row, the gathered sequence becomes a code to form one specific amino acid, and when several codes are gathered, it can be converted into a single protein. Amino acids may consist of alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), parolysine (Ply), proline (Pro), glutamine (Gln), arginine (Arg), serine (Ser), threonine (Thr), selenocysteine (Sec), valine (Val), tryptophan (Trp), and tyrosine (Tyr).

Peptide may refer to a peptide or a polypeptide composed of amino acid sequences. Within each species, there is an immune system in order to remove foreign substances that are not derived from genetic information of that species, and in particular, there is an immunogenic peptide that can induce an immune response, among externally derived peptides. Mutations that have different genetic information from the original genetic information in the course of cancer development also generate these immunogenic peptides, which can bind to HLA I protein through a series of processes in the immune system. Furthermore, the immunogenic peptide may have a mutant amino acid sequence. The length of the amino acid may be 25 or less but is not limited thereto, and the amino acid may have various lengths.

Neoantigen refers to a peptide that induces an immune response. That is, the neoantigen may be an immunogenic peptide. Neoantigens may be induced by tumor cell specific mutations and may be expressed as epitopes of tumor cells. Hereinafter, the immunogenic peptide is named as a neoantigen for clarity of description.

T cell activity data is data measuring the immune response that occurs when T cell is stimulated by binding of a specific peptide sequence to a specific HLA allele. The T cell activity data is obtained as data such as an expression value of cytokine in cells and an expression value of an immune cell specific activation marker that are detected by immunogenicity measurement experimental methodology including multimer/tetramer and ELISPOT, and the result value may be classified into "Positive," "Positive High," "Positive Low," "Positive Intermediate," and "Negative."

A neoantigen determination device according to the embodiments of the present invention can determine a specific peptide of a target cancer tissue to be used for treatment of the target cancer tissue, as a neoantigen, by analyzing the peptide sequence of the target cancer tissue and an HLA allele sequence of the patient. In addition, among the peptides contained in the target cancer tissue, a neoantigen suitable as an antigen can be determined. Furthermore, an antibody acting on the determined neoantigen can be searched and used for treatment of the target cancer tissue of the patient.

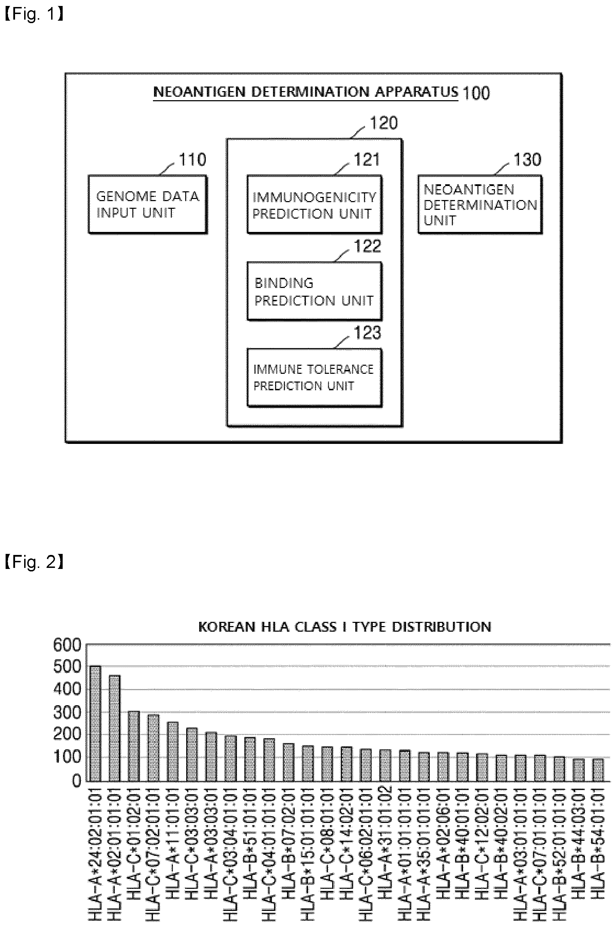

FIG. 1 is a block diagram of a neoantigen determination device 100 according to embodiments of the present invention.

The neoantigen determination device 100 is a device for determining a neoantigen for treating a disease existing in a cancer tissue, based on genomic data in the cancer tissue.

A genome data input unit 110 may receive a peptide sequence and an HLA allele sequence that are extracted from a cancer tissue. The peptide sequence may be one or more peptides included in the cancer tissue. The peptide sequence can be expressed as a two dimensional matrix to include sequences for peptides. The HLA allele sequence is expressed as a pseudo sequence or a full sequence. The HLA allele sequence may be expressed as an embedding vector in a specific size through a word embedding technique in which 1 to k amino acid units are set as one word but is not limited thereto. The HLA allele sequence may be expressed in various formats.

The genome data input unit 110 may separately calculate T cell activity data of the peptides or binding data between the peptides and the HLA allele, based on the peptide sequence and the HLA allele sequence.

The genome data input unit 110 may measure the T cell activity data for the peptides and calculate the T cell activity data for the peptides of cancer tissue by using a table or a database in which the measured data is recorded.

In this case, the HLA allele may be input by dividing the HLA allele sequence into a unit of 1 to k-mer and expressing the HLA allele sequence as a virtual word set, regardless of the entire sequence or the pseudo sequence.

The genomic data input unit 110 may measure the binding data related to binding affinity for all binding relations between the peptides and the HLA alleles and calculate the binding data between the peptides and the HLA alleles of the target cancer tissue by using a table or a database in which the measured binding data is recorded.

An immunogenicity prediction unit 121 may input the peptides and the HLA alleles as the T cell activity data and output a predictive value corresponding to immunogenicity to the peptides. The immunogenicity prediction unit 121 may output a first predictive value corresponding to immunogenicity to the peptides by using the T cell activity data and a model trained with as immunogenicity to the peptides. In this case, the peptide may be plural or singular. The T cell activity data may include those for the peptides sequence and/or those for the HLAs sequence.

A binding prediction unit 122 may input binding data for all binding relations between the peptides and the HLA alleles and output a second predictive value corresponding to the binding affinity to the peptides.

An immune tolerance prediction unit 123 may input the binding data for all binding relations between the peptides and the HLA alleles and output a third predictive value corresponding to the immune tolerance to the peptides.

A neoantigen determination unit 130 may output neoantigen information of the target cancer tissue by using a determination model trained based on the first to third prediction value. The neoantigen determination unit 130 may output whether a neoantigen has immunogenicity and binding affinity that can be used for treatment based on the first to third predictive value determined for each peptide among the first to third predicted values.

Based on these operations, the neoantigen determination device 100 may output whether the neoantigen can be utilized for treatment in consideration of the binding characteristics and immune characteristics of tumor cells or cancer cells included in the target cancer tissue. In addition, the neoantigen determination device 100 may output whether a neoantigen is recognized in consideration of the T cell activity data for the peptides of the target cancer tissue.

The neoantigen determination device 100 may be embodied to include at least one of a communication unit, an input unit, and an output unit, which are not shown, but is not limited thereto. The neoantigen determination device 100 may output data such as whether the neoantigen is recognized through the output unit. The neoantigen determination device 100 may receive a data outputting input through the input unit. The neoantigen determination device 100 may have a communication unit and communicate with external devices. At least one of the genome data input unit 110, the immunogenicity prediction unit 121, the binding prediction unit 122, the immune tolerance prediction unit 123, and the neoantigen determination unit 130 of the neoantigen determination device 100 may be embodied as in software or hardware. At least one of the immunogenicity prediction unit 121, the binding prediction unit 122, the immune tolerance prediction unit 123, and the neoantigen determination unit 130 may be implemented as one component.

The target cancer tissue of interest may be a cell engineered to express a single HLA class I allele or single HLA class II allele. The target cancer tissue may be human cells obtained from or derived from a plurality of patients. The target cancer tissue may include fresh or frozen tumor cells obtained from a plurality of patients. The target cancer tissue may include fresh or frozen tissue cells obtained from a plurality of patients. The target cancer tissue may include a peptide or peptides identified by using T-cell analysis.

The neoantigen determination device 100 may be trained with algorithms of the immunogenicity prediction unit 121, the binding prediction unit 122, the immune tolerance prediction unit 123, and the neoantigen determination unit 130 based on a plurality of the target cancer tissues. The neoantigen determination device 100 may be trained with at least one algorithm among the immunogenicity prediction unit 121, the binding prediction unit 122, the immune tolerance prediction unit 123, and the neoantigen determination unit 130, by using data related to proteomic sequences of the target cancer tissue, data related to HLA peptide sequences, binding data between the peptides and the HLA alleles, data related to transcripts related to the target cancer tissue, data related to genomes related to target cancer tissue, and the like.

At least one of the immunogenicity prediction unit 121, the binding prediction unit 122, the immune tolerance prediction unit 123, and the neoantigen determination unit 130 may be not independently constructed for each length of the peptides and may be constructed by recognizing peptides as a single word regardless of their length. At least one of the immunogenicity prediction unit 121, the binding prediction unit 122, the immune tolerance prediction unit 123, and the neoantigen determination unit 130 may embody the peptides as one word by using a word embedding technique.

Training data for at least one algorithm among the immunogenicity prediction unit 121, the binding prediction unit 122, the immune tolerance prediction unit 123, and the neoantigen determination unit 130 may also be input regardless of the length of the peptides. The neoantigen determination unit 130 may use an algorithm trained using deep learning.

The neoantigen determination device 100 may build a deep learning model classifying positive Y/negative N based on data. The neoantigen determination device 100 may use an additional neural network by fixing weights for the immunogenicity prediction unit 121, the binding prediction unit 122, the immune tolerance prediction unit 123, and the neoantigen determination unit 130. Through this, at least one of the immunogenicity prediction unit 121, the binding prediction unit 122, the immune tolerance prediction unit 123, and the neoantigen determination unit 130 may be embodied by using the immunogenicity data between the HLA allele and the peptide in the T cell activity data.

The immunogenicity prediction unit 121 may apply a word embedding technique to each amino acid of the peptides. The immunogenicity prediction unit 121 may extract feature values by applying CNN to a vector of peptides obtained by applying a word embedding technique. In this case, the feature values may be acquired through learning in various layers such as CNN. The immunogenicity prediction unit 121 may generate an algorithm, through a process of training positive or negative for immunogenicity of each peptide by applying a Gated Recurrent Unit (GRU) to the extracted feature values for the vector of peptides.

The binding prediction unit 122 may generate vectors by applying a word embedding technique to both the HLA alleles and the peptides and may extract feature values by applying CNN to a vector of the HLA allele and a vector of the peptide. The binding prediction unit 122 may apply the feature values to two neural networks to generate an encoder of the HLA allele and an encoder of the peptide and may generate an algorithm through a process of training positive or negative for a binding affinity by using the encoder of the HLA allele and the encoder of the peptide.

The training data used to generate the algorithm of the immune tolerance prediction unit 123 may be divided into positive and negative for immunogenicity. The immune tolerance prediction unit 123 may define a case where there is a binding ability to the HLA allele but no immunogenicity, which is therapeutic ability, as a tolerant peptide, and may define a peptide having a binding ability and immunogenicity as a non- tolerant peptide.

FIG. 2 is an exemplary graph of type information of HLA class I contained in cells of Koreans.

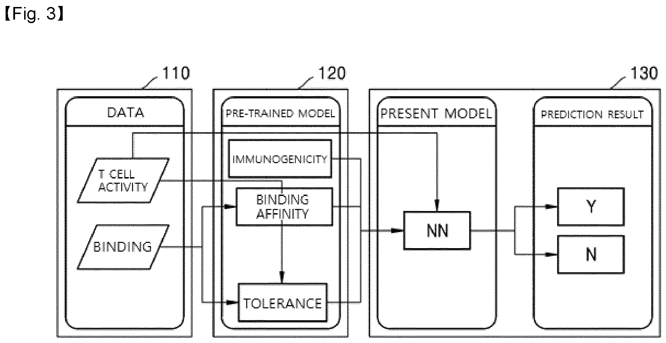

FIG. 3 is a block diagram of a neoantigen determination device 100 according to embodiments of the present invention.

As shown in FIG. 3, the neoantigen determination device 100 may generate T cell activity data and binding data.

The immunogenicity prediction unit of the neoantigen determination device 100 may output a first predictive value for immunogenicity by receiving the T cell activity data as an input.

The binding prediction unit may output a second predictive value for binding by receiving the T cell activity data and the binding data as an input.

The immune tolerance prediction unit may output a third predictive value for immune tolerance by receiving the T cell activity data and the binding data as an input.

A component 120 of the neoantigen determination device 100 may further include a prediction unit that predicts various factors other than the immunogenicity prediction unit, the binding prediction unit, and the immune tolerance prediction unit.

A component 130 of the neoantigen determination device 100 may determine whether the neoantigen can be used for treatment and output the determined result as one of Y and N.

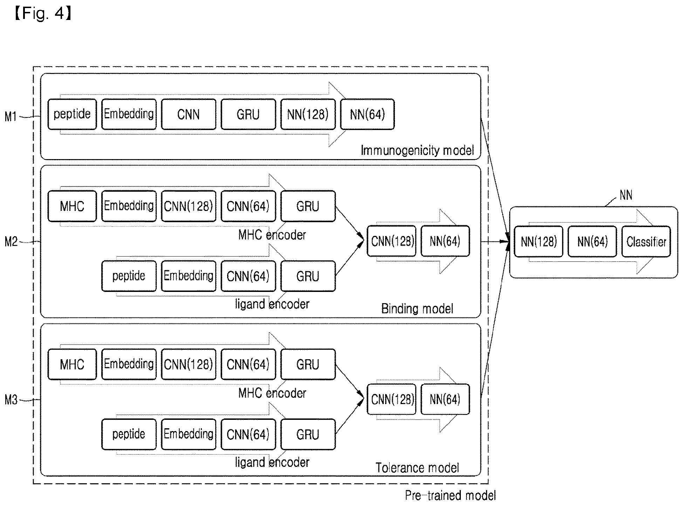

FIG. 4 is a block diagram for explaining input data and output data of a neoantigen determination device 100.

According to an embodiment of the present invention, the HLA allele sequence X1 and the peptide sequence X2 extracted from the target cancer tissue are used as input data, and N/Y corresponding to whether or not a neoantigen is recognized is output as output data.

In this case, the neoantigen determination device 100 may output N/Y corresponding to whether or not a neoantigen is present by using the immunogenicity prediction model M1, the binding prediction model M2, and the immune tolerance prediction model M3. Specifically, the neoantigen determination device 100 may output the N/Y corresponding to whether or not a neoantigen is present, by using the first predictive value output through the immunogenicity prediction model M1, the second predictive value output through the binding prediction model M2, and the third predictive value output through the immune tolerance prediction model M3 as inputs and further using a model NN trained to output N/Y corresponding to whether or not a neoantigen is present.

**[Table 1]**

| First Predictive Value | Second Predictive Value | Third Predictive Value | Output Value |
|---|---|---|---|
| Y | Y | Y | Y |
| Y | N | N | N |
| N | Y | N | N |
| N | N | N | N |

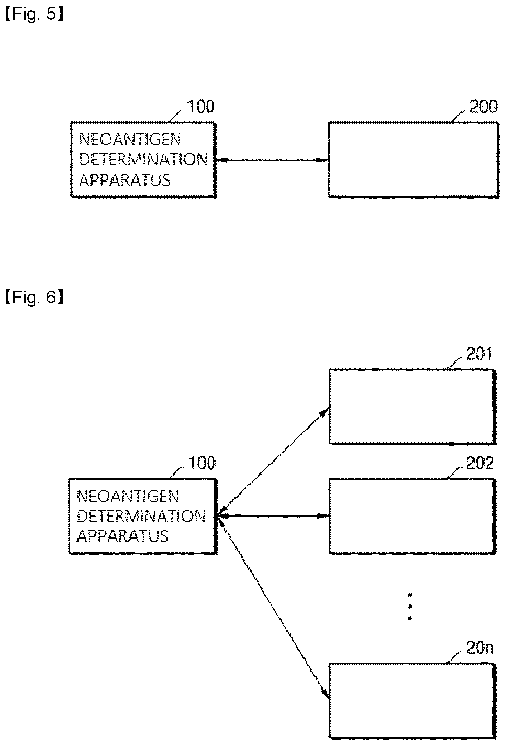

As shown in the table above, in a case where there is immunogenicity and no binding affinity between the peptide sequence and the HLA allele sequence of the target cancer tissue and in a case where there is a binding affinity and no immunogenicity, the neoantigen status may be output as N. In a case where there is both immunogenicity and a binding affinity between the peptide sequence and the HLA allele sequence of the target cancer tissue may be output as Y. FIGs. 5 to 7 are diagrams illustrating examples of a neoantigen determination system according to embodiments of the present invention.

As shown in FIG. 5, the neoantigen determination device 100 may receive genomic data of the cancer tissue from external electronic devices 200. The neoantigen determination device 100 may transmit the output information on whether the output is a neoantigen of the cancer tissue to the electronic device 200.

The electronic device 200 may be a computing device including one or more processors storing genomic data of cancer tissue. The electronic device 200 may be a device that outputs the genome data of cancer tissue. The electronic device 200 may be electrically connected to the neoantigen determination device 100 or may be connected to the neoantigen determination device 100 through a network to transmit and receive data.

The electronic device 200 may acquire and store genomic data of the cancer tissues of a plurality of samples several times. The neoantigen determination device 100 may sequentially output whether or not the neoantigen is a neoantigen to the genome data received from the electronic device 200.

As shown in FIG. 6, the neoantigen determination device 100 may receive data from a plurality of electronic devices 201, 202, ..., 20n and may transmit output data to a plurality of electronic devices 201, 202, ..., 20n.

The neoantigen determination device 100 may receive genome data from a plurality of electronic devices 201, 202, ..., 20n. The plurality of electronic devices 201, 202, ..., 20n may be managed by one or more entity.

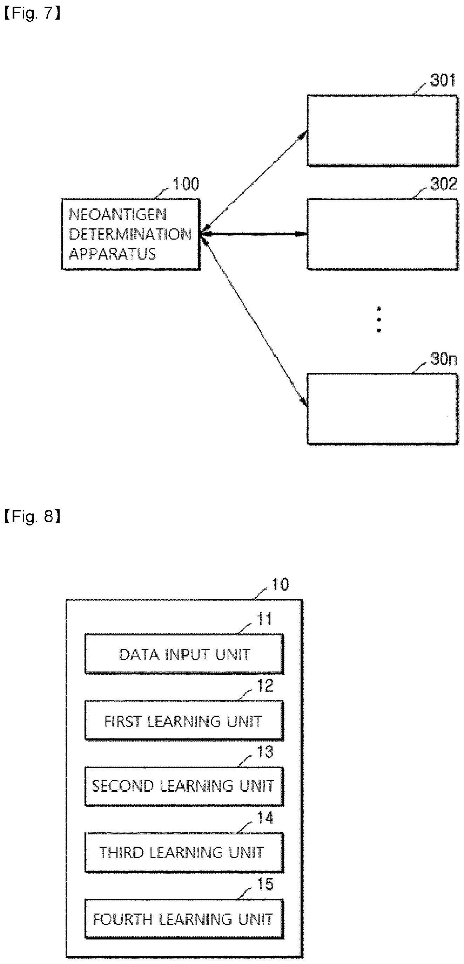

As shown in FIG. 7, the neoantigen determination device 100 may output data through the output unit of one or more terminal devices 301, 302, ..., 30n. The output data may be output through the output unit of the neoantigen determination device 100. The output data may be output through the output unit of the one or more terminal devices 301, 302, ..., 30n. The neoantigen determination device 100 may request a payment for a predetermined cost to one or more terminal devices 301, 302, ..., 30n as data related to the neoantigen is transmitted. One or more terminal devices 301, 302, ..., 30n may request neoantigen related information of peptides and HLA alleles that are included in cancer tissue. In response to the request, output data may be output.

FIG. 8 is a block diagram of a learning server 10 for learning an immunogenicity prediction model, a binding prediction model, an immune tolerance prediction model, and the like.

The learning server 10 may include a data input unit 11, a first learning unit 12, a second learning unit 13, a third learning unit 14, and a fourth learning unit 15.

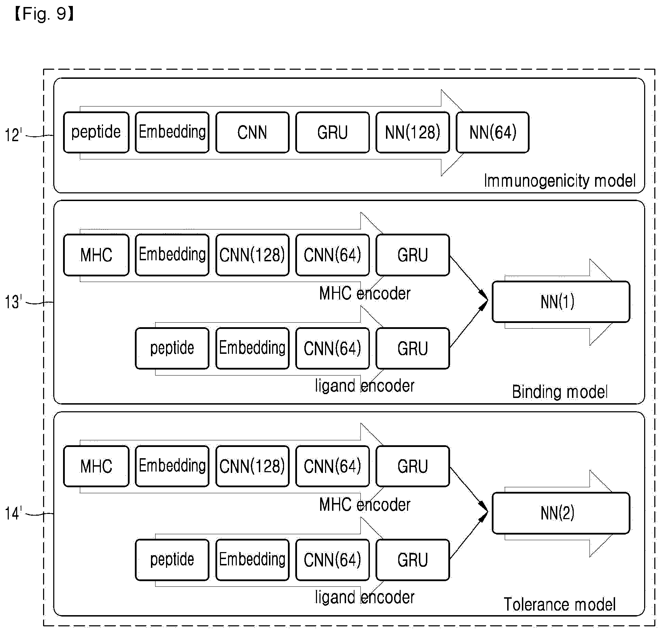

The first learning unit 12 trains and generates the immunogenicity prediction model, and the immunogenicity prediction model is trained with the T cell activity data of the peptide sequence or the HLA allele sequence and the immunogenicity of the peptide sequence as a training data set. As shown in a block 12' of FIG. 9, the immunogenicity prediction model trained by the first learning unit 12 processes the peptide sequence by a word embedding technique and learns by inputting the processed peptide sequence to layers of CNN, GRU, and NN.

The second learning unit 13 trains and generates the binding prediction model, and the binding prediction model is trained by using the binding data of the peptide sequence or the HLA allele sequence as an input and using the binding affinity between the peptide sequence and the HLA allele sequence as a training data set. As shown in a block 13' of FIG. 9, the binding prediction model trained by the second learning unit 13 processes the peptide sequence and the HLA allele sequence by a word embedding technique, learns by inputting the processed peptide sequence to layers of CNN and GRU, and learns by inputting the HLA allele sequence to layers of CNN, CNN, and GRU. The binding prediction model may be trained to train another model NN1 with a predictive value for a binding affinity to the peptide sequence and a predictive value for a binding affinity to the HLA allele sequence and to finally output a predictive value for immune tolerance to the target cancer tissue.

The third learning unit 14 trains and generates the immune tolerance prediction model, and the immune tolerance prediction model is trained by using the peptide sequence or the HLA allele sequence as an input and using the immune tolerance between the peptide sequence and the HLA allele sequence as a training data set. As shown in a block 14' of FIG. 9, the immune tolerance prediction model trained by the third learning unit 14 processes the peptide sequence and the HLA allele sequence by a word embedding technique, learns by inputting the processed peptide sequence to layers of CNN and GRU, and learns by inputting the HLA allele sequence to layers of CNN, CNN, and GRU. The immune tolerance prediction model may be trained to train another model NN2 with a predictive value for an immune tolerance to the peptide sequence and a predictive value for an immune tolerance to the HLA allele sequence and to finally output a predictive value for immune tolerance to the target cancer tissue.

The learning server 10 may transmit the learning models generated by the first to third learning units 12, 13, and 14 to the neoantigen determination device 100. Through this process, the algorithms of the immunogenicity prediction unit 121, the binding prediction unit 122, and the immune tolerance prediction unit 123 of the neoantigen determination device 100 may be periodically updated. The device described above may be embodied as a hardware component, a software component, and/or a combination of the hardware component and the software component. For example, the device and components described in the embodiments may be embodied using one or more general purpose or special purpose computers such as a processor, a controller, an arithmetic logic unit (ALU), a digital signal processor, a microcomputer, a field programmable gate array (FPGA), a programmable logic unit (PLU), a microprocessor, or any other device capable of executing and responding to instructions. The processing device may execute an operating system (OS) and one or more software applications running on the operating system. In addition, the processing device may access, store, manipulate, process, and generate data in response to execution of the software. Although one processing device is described as being used in the system for convenience of explanation and understanding, one of ordinary skill in the art will recognize that the processing device may include a plurality of processing elements and/or a plurality types of processing elements. For example, the processing device may be embodied to include a plurality of processors or to include one processor and one controller. In addition, the processing device may be embodied as other processing configurations such as parallel processors.

Software may include a computer program, code, an instruction, or a combination of one or more of these elements and may be configured to operate as desired or to instruct the processing device independently or collectively. The software and/or data may be permanently or temporarily embodied as any kind of a machine, component, physical device, virtual equipment, computer storage medium or device, or transmitted signal wave, in order to be interpreted by the processing device or to provide instructions or data to the processing device. The software may be distributed over computer systems connected through networks and stored or executed in a distributed manner. Software and data may be stored in one or more computer-readable recording media.

The method according to the embodiment may be embodied in a form of program instructions that can be executed through various computer means and be recorded in a computer-readable medium. The computer-readable medium may include each or a combination of program instructions, data files, data structures, and others. The program instructions recorded on the medium may be specially designed and configured for the embodiment or may be known and available to those skilled in the art of computer software. Examples of the computer-readable recording medium include magnetic media such as hard disk, floppy disk, and magnetic tape, optical media such as CD-ROM and DVD, magneto-optical media such as floptical disk, and hardware devices specially configured to store and execute instructions of a program such as ROM, RAM, flash memory, and the like. Examples of program instructions include machine language codes such as those generated by a compiler and high level language codes that can be executed by a computer using an interpreter or the like. The hardware devices described above may be configured to operate as one or more software modules to perform the operations of the embodiments and vice versa.

As described above, although the embodiments have been described with reference to the limited embodiments and drawings, various modifications and variations are possible from the above description by those skilled in the art. For example, although the described techniques are performed in an order different from the described method, and/or the described components of the system, structure, device, and circuit are combined in a different form from the described method or are substituted by other components or equivalents, those modifications may achieve an appropriate result described in the present invention.

Therefore, other embodiments, and equivalents to the appending claims are also within the scope of the following claims.

## Claims

1. A method of predicting a neoantigen by using a peptide sequence and an HLA allele sequence, the method comprising the steps of:
receiving a peptide sequence and an HLA allele sequence both extracted from a target cancer tissue as an input;
outputting a first prediction value that predicts immunogenicity of the peptide sequence, by acquiring T cell activity data from the peptide sequence and inputting the T cell activity data into an immunogenicity prediction model;
outputting a second prediction value that predicts a binding affinity of the peptide sequence and the HLA allele sequence, by acquiring binding data from the HLA allele sequence and inputting the binding data into a binding prediction model;
outputting a third prediction value that predicts immune tolerance of the target cancer tissue, by inputting the T cell activity data and the binding data to an immune tolerance prediction model; and
generating neoantigen information of a target cell by using the T cell activity data and the first to third prediction value.

2. The method of claim 1, wherein at least one of the immunogenicity prediction model, the binding predictive model, and the immune tolerance prediction model is trained by a machine learning algorithm, based on a training data set including a peptide sequence and an HLA allele sequence present in a plurality of target cancer tissues.

3. The method of claim 2, wherein the target cancer tissue includes a cell engineered to express a single HLA class I allele or single HLA class II allele.

4. The method of claim 2, wherein the target cancer tissue includes a human cell obtained from or derived from a plurality of patients.

5. The method of claim 2, wherein the target cancer tissue includes a fresh or frozen tumor cell obtained from a plurality of patients.

6. The method of claim 2, wherein the target cancer tissue includes a fresh or frozen tissue cell obtained from a plurality of patients.

7. The method of claim 2, wherein the target cancer tissue includes a peptide identified by using T-cell analysis.

8. The method of claim 2, wherein the training data set includes at least one of data related to a proteomic sequence related to the target cancer tissue, data related to an HLA peptide sequence related to the target cancer tissue, binding data between a peptide and an HLA allele that are related to the target cancer tissue; data related to a transcriptome related to the target cancer tissue; and data related to a genome related to the target cancer tissue.

9. The method of claim 1, wherein the immunogenicity prediction model is a model trained with the T cell activity data from the peptide sequence as an input and the immunogenicity of the peptide sequence as an output.

10. The method of claim 1, wherein the binding prediction model is a model trained with the binding data from the HLA allele sequence and the peptide sequence as an input and the binding affinity of the peptide sequence and the HLA allele sequence as an output.

11. The method of claim 1, wherein the immune tolerance prediction model is a model trained with the T cell activity data from the peptide sequence and the HLA allele sequence, and the binding data from the HLA allele sequence and the peptide sequence as an input and the immune tolerance between the peptide sequence and the HLA allele sequence.

12. A computer program stored in a computer-readable storage medium for executing the method of any one of claim 1 to 11 by using a computer.
